# DEMANDE DE BREVET EUROPEEN

(11) **EP 4 190 405 A1**
(43) Date de publication de la demande: **07.06.2023**
(21) Numéro de dépôt: 22210719.5
(22) Date de dépôt: 01.12.2022
(51) Int. Cl.: A61Q 19/08, A23L 3/28, A61K 8/96

(54) **PROCÉDÉ DE PRÉPARATION D EAU EN VUE D APPLICATION CUTANÉE, PROCÉDÉ DE SOINS CUTANÉS ESTHÉTIQUES**

(30) Priorité: 01.12.2021 FR 2112799
(71) Demandeur: Rosati, Eric, 75008 Paris (FR)
(72) Inventeur: Rosati, Eric, 75008 Paris (FR)
(74) Mandataire: Lecomte & Partners

(57) **Abrégé**

La présente invention concerne un procédé de préparation d'eau en vue d'application cutanée, notamment pour des soins esthétiques, comprenant les étapes suivantes : (a) mise à disposition d'une quantité d'eau ; (b) traitement de la quantité d'eau, le procédé est remarquable en ce que l'étape (b) comprend une application d'un champ électromagnétique à une ou plusieurs fréquences comprises entre 10 et 40 GHz. Procédé de soins cutanés esthétiques comprenant l'étape (c) d'application d'eau préparée sur la peau (22), remarquable en ce que l'eau préparée est obtenue par le procédé de préparation d'eau.

## Description

### Domaine technique

La présente invention concerne le domaine des soins esthétiques, plus particulièrement le domaine des techniques de soins cutanés reposant sur le principe de la biophysique et utilisant des ondes électromagnétiques.

### Art antérieur

La peau humaine est constituée de trois différentes couches, i.e. l'épiderme, le derme et l'hypoderme. Le derme de la peau contient des fibroblastes, ces derniers sont des cellules dans lesquels des fibres élastines et de collagène sont synthétisées. Ces deux fibres sont les principaux constituants du derme qui lui donnent son élasticité et sa souplesse.

Les soins esthétiques ou thérapeutiques reposant sur le principe de la biophysique sont connus de l'état de la technique. En effet, l'objectif de ces soins consiste à stimuler la peau au moyen d'un système s'opérant par biorésonance afin de restaurer le derme de celle-ci, cette stimulation pouvant être réalisée au moyen de fréquences électromagnétiques transitées par un support fluide, notamment de l'eau.

L'effet des champs électromagnétiques sur des molécules d'eau (H₂O) a été démontré, notamment dans la publication « Changes in the State of Water, Induced by Radiofrequency Electromagnetic Fields » (1995) Fesenko, E.E. et Gluvstein, A. qui enseigne que lorsqu'une quantité d'eau est sollicitée par un champ électromagnétique à 36GHz, les propriétés diélectriques et conductrices de l'eau changent.

Le document de brevet JPH 04235904 divulgue la préparation d'eau par application d'ondes électromagnétiques de basse fréquence pour des applications cosmétiques.

Le document de brevet publié US 2016/0008620 A1 divulgue un dispositif thérapeutique permettant la stimulation du cerveau par le moyen d'ondes électromagnétiques de basse fréquence afin de soigner l'état morbide dudit cerveau. En effet, la régulation de l'état d'une cellule comprise dans le derme par l'ADN est un principe connu en neurosciences, cette régulation pouvant être réalisée par le cerveau si ce dernier est stimulé par un moyen thérapeutique. Cependant, aucun lien entre le cerveau et les cellules comprises dans le derme de la peau n'a été divulgué dans le document US 2016/0008620 A1.

Le document de brevet publié EP 1 747 798 A2 divulgue un système de génération de champs électromagnétiques dans un bain d'eau. Le document révèle en outre que l'immersion d'un patient dans cette eau chargée électromagnétiquement permet de traiter les maladies de sa peau. Cependant, la fréquence du champ électromagnétique généré n'est pas divulguée.

### Résumé de l'invention

La présente invention a pour objectif de pallier au moins un des inconvénients de l'état de la technique susmentionné. Plus particulièrement, l'invention a pour objectif de procurer de meilleurs soins cutanés esthétiques, en particulier de régénérer de manière efficace les fibroblastes de la peau.

À cet effet, l'invention a pour objet un procédé de préparation d'eau en vue d'application cutanée, notamment pour des soins esthétiques, comprenant les étapes suivantes : (a) mise à disposition d'une quantité d'eau ; (b) traitement de la quantité d'eau, le procédé étant remarquable en ce que l'étape (b) de traitement de la quantité d'eau comprend une application d'un champ électromagnétique à une ou plusieurs fréquences comprises entre 10 et 40 GHz.

Selon un mode de réalisation, le champ électromagnétique présente une densité de puissance d'au moins 2 mW/cm2 en tout point d'au moins 80% de la quantité d'eau.

Selon un mode de réalisation, l'application du champ électromagnétique à l'étape (b) dure au moins 10 minutes pour toute unité de volume de la quantité d'eau.

L'invention a pour objet un procédé de soins cutanés esthétiques comprenant l'étape (c) d'application d'eau préparée sur la peau, remarquable en ce que l'eau préparée est obtenue par le procédé de préparation d'eau.

Selon un mode de réalisation, l'étape (c) dure au moins 20 minutes.

Selon un mode de réalisation, l'étape (b) est réalisée préalablement à l'étape (c) et comprend une application d'un générateur de champ électromagnétique contre un récipient contenant la quantité d'eau.

Avantageusement, l'invention permet la modification de la structure moléculaire de l'eau permettant aux différentes fréquences vibratoires (ondes électromagnétiques) de pouvoir pénétrer dans au moins une cellule pour en améliorer la fonction primaire. De façon avantageuse, la stimulation de la cellule permet une régénération de son collagène via une action sur les fibroblastes.

L'invention repose au moins en partie sur le principe du biofeedback, i.e. un ensemble de techniques principalement relatives à la bioélectricité pour la mesure de fonctions organiques reposant sur la visualisation au moyen d'appareils électriques ou des signaux physiologiques d'un sujet conscient de ces mesures.

### Description des dessins

[Fig 1] représente une vue schématique de deux récipients contenant des molécules d'eau avant et après leurs traitements suivant le procédé de préparation d'eau ;
[Fig 2] est une représentation schématique du procédé de préparation d'eau et du procédé de soins esthétiques en vue d'application cutanée selon l'invention ;
[Fig 3] est une illustration de l'utilisation du procédé de soins cutanés esthétiques sur un sujet.

### Description détaillée

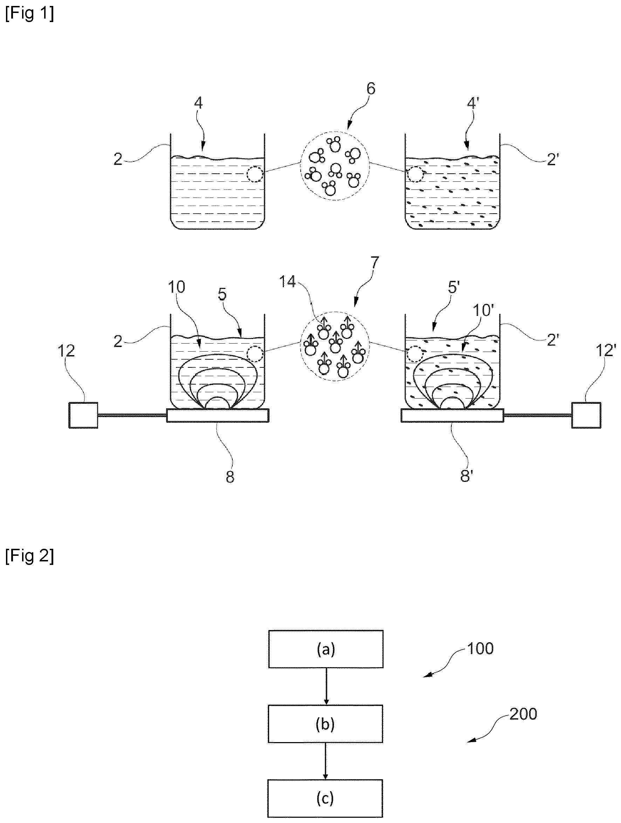

La partie supérieure de la figure 1 représente une vue schématique de deux récipients 2, 2' contenant des molécules d'eau (*H₂O*) avant leurs traitements suivant un procédé de préparation d'eau. La partie inférieure de la figure 1 représente une vue schématique des deux récipients 2, 2' après leurs traitements suivant le procédé de préparation d'eau.

En référence à la partie supérieure de la figure 1, le récipient 2 contient de l'eau 4, tandis que le récipient 2' contient une substance crémeuse 4' comprenant des molécules d'eau et qui peut être un masque d'argile, d'alginate, une crème, un sérum ou tout autre masque ou produit disponible sur le marché et communément utilisé à des fins esthétiques ou cosmétiques, préférentiellement pour le visage.

Une section partielle agrandie 6 est illustrée au niveau de la partie centrale entre les deux récipients 2 et 2', la section 6 représente les molécules d'eau comprises dans l'eau 4 et dans la substance crémeuse 4', ces derniers sont dans un état de repos et orientés d'une façon aléatoire.

En référence à la partie inférieure de la figure 1, les récipients 2 et 2' sont positionnés par au-dessus et en contact direct d'un générateur de champ électromagnétique 8, 8' appliquant un champ électromagnétique 10, 10'.

À cet égard, les récipients 2, 2' sont préférentiellement composés d'un matériau isolant et favorisant la diffusion du champ électromagnétique 10, 10', tel que le verre ou le plastique par exemple.

De préférence, le générateur de champ électromagnétique 8, 8' comprend au moins une bobine et un noyau, aptes à émettre un champ électromagnétique 10, 10' à des fréquences supérieures à 5 GHz et/ou inférieures à 50 GHz, préférentiellement comprises entre 10 et 40 GHz, et encore plus préférentiellement comprises entre 18 et 22 GHz. Lesdites fréquences peuvent être utilisées en combinaison ou séparément.

Le générateur de champ électromagnétique 8, 8' peut également comprendre un circuit électronique, par exemple du type LRC, c'est-à-dire un circuit électrique fermé comprenant une inductance L, une capacité C et une résistance R, apte à générer un signal électrique parcourant l'au moins une bobine. Ce circuit électronique peut toutefois être extérieur au générateur de champ électromagnétique et relié électriquement audit générateur.

Le générateur de champ électromagnétique 8, 8' peut être alimenté électriquement par une alimentation électrique 12, 12', cette dernière peut être une batterie de stockage ou un chargeur branché à une prise domestique. Le générateur 8, 8' comprend une face d'émission correspondant à sa face supérieure en contact avec la face inférieure du récipient 2, 2' tel qu'illustré dans la figure 1.

La stimulation par application du champ électromagnétique 10, 10' sur l'eau 4 ou sur la substance crémeuse 4' pendant une durée prédéfinie permet d'obtenir une eau structurée 5 ou une substance crémeuse structurée 5', ces dernières sont distinguées par leurs molécules d'eau qui s'orientent globalement en fonction de la direction de propagation du champ électromagnétique appliqué.

Préférentiellement, la durée de stimulation est d'au moins 10 minutes et plus préférentiellement d'au moins 15 minutes et peut aller jusqu'à quelques dizaines d'heures.

Une section partielle agrandie 7 illustre les molécules d'eau de l'eau structurée 5 ou de la substance crémeuse 5', les molécules sont orientées verticalement suivant la direction de propagation du champ électromagnétique 10, 10' appliqué par le générateur de champ électromagnétique 8, 8'.

Les molécules telles qu'illustrées dans la section partielle agrandie 7, comportent une information vibratoire 14, également appelée message fréquentiel 14, représenté par une flèche ayant la direction de propagation du champ électromagnétique 10, 10' et orientée suivant le sens d'application dudit champ électromagnétique 10, 10'. En effet, le temps minimum nécessaire pour permettre aux molécules d'eau de porter le message fréquentiel 14 est d'environ 10 minutes.

La figure 2 représente schématiquement un procédé 100 de préparation d'eau en vue d'application cutanée, notamment pour des soins esthétiques visant à hydrater la peau et plus spécifiquement visant à régénérer de manière efficace les fibroblastes compris dans le derme de la peau. La figure 2 représente également un procédé 200 de soins cutanés esthétiques.

En référence aux figures 1 et 2, le procédé 100 de préparation d'eau comprend une étape (a) de mise à disposition d'une quantité d'eau 4 ou de substance crémeuse 4', cette dernière sera considérée comme étant une quantité d'eau 4, 4' pour le reste de la description.

Le procédé 100 comprend également une étape (b) de traitement de la quantité d'eau 4, 4' en application du champ électromagnétique 10, 10' par l'appareil 8, 8'. De préférence, le champ électromagnétique 10, 10' a une ou plusieurs fréquences, ces dernières sont en fait de très grandes fréquences supérieures à 5 Gigahertz et/ou inférieures à 50 GHz, préférentiellement comprises entre 10 et 40 GHz, et encore plus préférentiellement comprises entre 18 et 22 GHz. Lesdites très grandes fréquences peuvent être utilisées en combinaison ou séparément.

De plus, le champ électromagnétique 10, 10' présente une densité de puissance qui est proportionnelle au produit du champ électrique par le champ magnétique, la densité de puissance de ce dernier est d'au moins 0,5 mW/cm2 en tout point d'au moins la moitié de la quantité d'eau 4, 4'. De préférence, la densité de puissance du champ électromagnétique 10, 10' est d'au moins 2 mW/cm2 en tout point d'au moins 80% de la quantité d'eau 4, 4'. Cependant, il a été observé qu'au-delà de 20 mW/cm2, les effets avantageux dus à la stimulation des molécules d'eau par le champ magnétique 10, 10' sont moins présents.

Préférentiellement, la durée de l'étape (b) de traitement de la quantité d'eau 4, 4' est d'au moins 10 minutes et plus préférentiellement d'au moins 30 minutes et peut aller jusqu'à quelques dizaines d'heures.

Le procédé 200 de soins cutanés esthétiques utilise les étapes (a) et (b) du procédé 100 de préparation d'eau, et comprend en outre une étape (c) d'application d'eau préparée 5, 5' sur la peau, cette dernière correspond à l'eau structurée 5 ou à la substance crémeuse 5' structurée de la figure 1.

De préférence l'eau préparée 5 est mélangée à de l'argile, alginate, crème, sérum ou tout autre produit disponible sur le marché et communément utilisé à des fins de préparation de masque esthétique, ou la substance crémeuse 5' est directement appliquée sur la peau du sujet quelques heures après la fin du traitement à l'étape (b).

De préférence, l'application de la substance crémeuse 5' sur la peau est réalisée après un repos de ladite substance crémeuse 5' qui dure au moins 18 heures après la fin de l'étape (b), et plus préférentiellement après au moins 25 heures. La durée de repos des molécules d'eau stimulées dépend du volume de la quantité de la substance crémeuse 5' électromagnétiquement traitée. Préférentiellement, l'application à l'étape (c) est réalisée au moyen d'un roller sur la peau du sujet.

L'étape (b) est réalisée préalablement à l'étape (c). La durée de l'étape (c) d'application de l'eau préparée 5, 5' sur la peau est d'au moins 20 minutes et préférentiellement d'au moins 30 minutes. Cependant, la durée d'application sur la peau peut durer plusieurs dizaines d'heures. Au bout de ladite durée d'application, une succession de réactions en intracellulaire vont s'opérer, ces réactions rentrent dans le domaine des neurosciences et ne font pas partie du procédé de soins cutanés esthétiques revendiqué.

À cet égard, pendant ou après l'application de l'eau préparée 5, 5' sur la peau, une réception du message fréquentiel 14 est opérée, ce dernier est porté par les molécules d'eau comprises dans l'eau préparée 5, 5' par au moins une cellule comprise dans le derme de la peau du sujet. En effet, le message fréquentiel 14 est acheminé à l'intérieur de la cellule par un fonctionnement biologique appelé communication cellulaire qui peut être similaire à un mécanisme de soupape.

Le message fréquentiel 14 reçu par la cellule déclenche deux réactions, une première réaction de correction de la cellule distinguée par une restructuration des molécules d'eau contenues dans ladite cellule. En effet, les cellules du corps humain contiennent 99% d'eau sur le plan moléculaire et le message fréquentiel 14 va créer une réaction au sein de l'ADN de la cellule lui permettant de modifier le fonctionnement de celle-ci en rétablissant l'équilibre naturel de ladite cellule, i.e. stimulation des fibroblastes dans un premier temps résultant en une accélération de la reproduction de collagène.

En effet, la différence entre la tension à l'intérieur de la cellule et la tension entre les cellules est appelée potentiel de tension, ce dernier est responsable du métabolisme physiologique sain des cellules. Avantageusement, lors de la sollicitation d'une membrane cellulaire par le message fréquentiel 14, ce dernier est perçu comme une information de recalibrage de la fonction primaire de la cellule, causant ainsi une reprogrammation des fibroblastes.

En parallèle de la première réaction de correction de la cellule, une deuxième réaction est opérée et dans laquelle le message fréquentiel 14 peut être transmis directement au cerveau pour ainsi le stimuler et créer une réaction résultant par une action correctrice de la cellule, celle-ci est similaire à la première réaction de correction de la cellule, mais distinguée par le fait que c'est le cerveau qui donne l'ordre à la cellule de régénérer le collagène par stimulation de l'ADN et non pas par le message fréquentiel 14 directement.

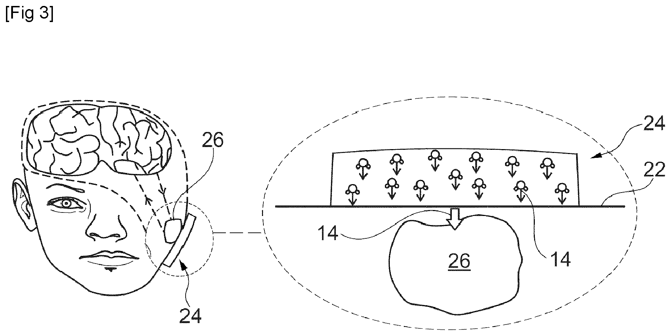

La figure 3 est une illustration de l'utilisation du procédé de soins cutanés esthétiques sur un sujet, préférentiellement sur la peau 22 de son visage. La figure 3 comprend à sa partie droite une section partielle agrandie et orientée horizontalement de façon à faciliter sa description.

En référence à la partie gauche de la figure 3, un support 24 est illustré comme étant positionné sur le visage du sujet, mais il peut être également positionné sur sa poitrine ou ses mains ou toute autre partie de son corps dans laquelle des résultats avantageux peuvent être obtenu grâce à l'utilisation du procédé de soins cutanés esthétiques.

De plus, la partie gauche de la figure 3 illustre une vue partielle schématisant les réactions intracellulaires décrites ci-dessus, notamment entre l'au moins une cellule 26 du derme et le cerveau du sujet.

En référence à la partie droite de la figue 3, l'étape (c) d'application d'eau préparée sur la peau est préférablement réalisée au moyen du support 24 comprenant des molécules d'eau, ledit support 24 peut correspondre à l'eau préparée 5 ou à la substance crémeuse préparée 5' de la figure 1, qui suite à l'application du champ électromagnétique, les molécules d'eau comprises dans le support 24 comprennent le message fréquentiel 14.

Le support 24 est en contact direct avec la peau 22. Avantageusement, cela permet de transmettre efficacement le message fréquentiel 14 à l'au moins une cellule 26 de la peau 22.

Le support 24 peut être un récipient comprenant de l'eau stimulée ou une substance crémeuse traitée, ou bien ça peut être un patch comprenant des molécules d'eau dans sa composition et ayant préalablement été stimulé électromagnétiquement selon le procédé de préparation d'eau de l'invention.

En effet, le support 24 peut être tout autre élément configuré pour pouvoir être appliqué sur la peau 22 du sujet et ayant été traité selon le procédé de préparation d'eau de l'invention. Préférentiellement, le support 24 était au repos au moins 18h avant son application.

Des résultats expérimentaux suite à l'utilisation du procédé de soins cutanés esthétiques sur la peau de différents sujets ont démontré plusieurs effets avantageux, notamment avec l'utilisation d'un champ électromagnétique ayant au moins une fréquence comprise entre 10 et 40 GHz. Par exemple, la détoxification de l'épiderme, un teint plus clair et lumineux, un épiderme repulpé, une hydratation de la peau en profondeur et une diminution des signes de fatigue particulièrement sur le visage des sujets.

**[Table 1]**

| | | Masque (stimulé) | Témoin (non stimulé) |
|---|---|---|---|
| Critère 1 : Hydratation | Évolution de l'indice mesuré par cornéométrie | +4,2% | +3% |
| Critère 2 : Analyse des pores | Évolution des moyennes des aires de pores | -39% | -30,1 % |
| | Évolution des nombres de pores | -32% | -29,3% |
| Critère 3 : Teint | Évolution de la clarté | +1,6% | +0,3% |
| | Évolution du rouge | +7,3% | +1,9% |
| Critère 4 : Relief joue | Évolution du micro relief | -12% | -6% |
| | Évolution du volume du relief | -94,3% | -48,3% |

Le tableau ci-dessus représente un résumé des résultats expérimentaux réalisés sur plusieurs sujets, précisément sur sept sujets différents.

Les expériences réalisées consistent à appliquer sur chaque sujet simultanément un masque contentant de l'eau stimulée par un champ électromagnétique et un masque identique mais dont l'eau n'est pas stimulée et qu'on appelle témoin. Par exemple, le masque peut être la substance crémeuse structurée 5' de la figure 1 qui est préparée suivant le procédé de préparation d'eau, alors que le témoin peut être la substance crémeuse 4' qui n'a subi aucune stimulation.

Le masque et le témoin ont été appliqués sur le visage des différents sujets suivant le procédé de soins cutanés esthétiques de la présente invention, notamment en appliquant par exemple le masque sur un côté du visage et le témoin sur l'autre.

L'application du masque et du témoin a été réalisée sur les sujets une fois par semaine pendant cinq semaines afin d'évaluer les critères 2 à 4 de la table 1, notamment l'analyse des pores, le teint et le relief de la joue. Alors que le premier critère d'hydratation a été évalué sur trois semaines uniquement.

En référence au premier critère d'hydratation de la table 1, son évaluation a été réalisée suite à la mesure d'un indice obtenue au moyen d'un outil appelé Cornéomètre^{®} disposant d'une sonde lui permettant de mesurer une capacitance dans un milieu diélectrique, notamment la peau, pour exprimer la capacitance en indice.

L'indice mesuré par cornéométrie sur la peau du visage d'un des sept sujets était de 63,32 avant l'application du témoin et de 65,20 au bout de la troisième semaine, démontrant alors une évolution de l'ordre de 3%. Parallèlement, l'indice était de 68,70 avant l'application du masque et de 71,61 après la même période, l'évolution cette fois est de 4,60%.

De façon avantageuse, le masque dont les particules d'eau sont stimulées électromagnétiquement à des fréquences comprises dans la plage 10-40 GHz a permis de significativement améliorer l'hydratation de la peau du sujet.

Le deuxième critère d'analyse de pores de la table 1 est évalué suivant les mesures de deux aspects, un premier aspect concernant l'aire des pores et qui est évalué suivant la moyenne des aires des différents pores compris dans une surface de peau précise, et un autre aspect quantifiant ces pores. Les deux aspects sont mesurés au moyen d'un outil appelé « VISIA-CR » capable de réaliser des imageries faciales numériques.

On observe donc sur un des sujets après cinq semaines que le nombre et l'aire des pores ont diminués de manière plus conséquente avec le masque actif qu'avec le témoin.

Le teint est le troisième critère tel que représenté dans la table 1, celui-ci est évalué suivant l'évolution de la clarté et de la rougeur de la peau des sujets, ces derniers sont tous les deux mesurés par le moyen d'un spectromètre. En effet, les résultats montrent que la peau est plus claire de 1,60% et moins rouge de 7,30% après la cure en utilisant le masque au bout de cinq semaines, cette évolution est moindre après l'utilisation du témoin.

Le quatrième critère du relief de la joue de la table 1 est évalué par la mesure de l'évolution du micro relief ainsi que par l'évolution du volume du relief. En effet, le micro relief correspond à la rugosité de la peau, donc plus la peau est lisse et plus la hauteur des micro reliefs est moindre, c'est justement le résultat qu'a été obtenu avec l'utilisation du masque sur un sujet, on a constaté une diminution du micro relief de l'ordre de 12%, alors qu'avec l'utilisation du témoin, la diminution n'était que de 6%.

Le volume du relief de la joue a été mesuré en mm³, cette mesure a également connu une importante diminution, notamment entre l'utilisation du témoin et du masque, ce dernier a permis à un des sujets de diminuer le volume des reliefs sur sa peau à plus 46% en moins comparé avec l'utilisation du témoin non stimulé.

Avantageusement, l'utilisation du procédé de soins cutanés esthétiques sur la peau d'un sujet permettra de stimuler les récepteurs du derme sur tout le visage et ainsi activer une stimulation globale des méridiens de celui-ci. Par voie de conséquence, cela permettra de réguler l'état physiologique du corps du sujet en intracellulaire.

Les mesures de l'invention sont avantageuses en outre en ce qu'elles permettent de maintenir l'homéostasie du corps humain et de non pas seulement régénérer les fibroblastes. En fait, en fonction des zones du visage ou du cou traitées par le procédé de soins cutanés de l'invention, des actions bénéfiques pour différents organes peuvent avoir lieu. Par exemple, un traitement appliqué sur les joues permettra de soulager l'estomac.

Avantageusement, en cas d'inflammation ou de morbidité de la cellule, la stimulation de cette dernière par le procédé de soins cutanés esthétiques de l'invention permettra de corriger l'état morbide de l'organe et de soigner son état inflammatoire.

## Revendications

1. Procédé (100) de préparation d'eau en vue d'application cutanée, notamment pour des soins esthétiques, comprenant les étapes suivantes :
(a) mise à disposition d'une quantité d'eau (4, 4') ;
(b) traitement de la quantité d'eau (4, 4') ;
**caractérisé en ce que** l'étape (b) de traitement la quantité d'eau (4, 4') comprend une application d'un champ électromagnétique (10, 10') à une ou plusieurs fréquences comprises entre 10 et 40 GHz.

2. Procédé (100) selon la revendication 1, **caractérisé en ce que** le champ électromagnétique (10, 10') présente une densité de puissance d'au moins 2 mW/cm² en tout point d'au moins 80% de la quantité d'eau (4, 4').

3. Procédé (100) selon l'une des revendications 1 et 2, **caractérisé en ce que** l'application du champ électromagnétique (10, 10') à l'étape (b) dure au moins 10 minutes pour toute unité de volume de la quantité d'eau (4, 4').

4. Procédé (200) de soins cutanés esthétiques comprenant l'étape suivante :
(c) application d'eau préparée (5, 5') sur la peau (22) ;
**caractérisé en ce que**
l'eau préparée (5, 5') est obtenue par le procédé (100) selon l'une des revendications 1 à 3.

5. Procédé (200) selon la revendication 4, **caractérisé en ce que** l'étape (c) dure au moins 20 minutes.

6. Procédé (200) selon l'une des revendications 4 et 5, **caractérisé en ce que** l'étape (b) est réalisée préalablement à l'étape (c) et comprend une application d'un générateur de champ électromagnétique contre un récipient contenant la quantité d'eau.
